# EUROPEAN PATENT APPLICATION

(11) **EP 1 570 818 A1**
(43) Date of publication of application: **07.09.2005**
(21) Application number: 04445021.1
(22) Date of filing: 01.03.2004
(51) Int. Cl.: A61F 5/01, A61F 13/10

(54) **Support bandage**

(71) Applicant: CAMP SCANDINAVIA AB, 254 67 Helsingborg (SE)
(72) Inventor: Smits, Jan F.A., 5709 MP Helmond (NL)
(74) Representative: Akerman, Marten Lennart

(57) **Abstract**

The invention relates to a support bandage (1) for treatment and pain relief of repetitive strain injuries, typically tennis elbow (epicondylitis) and the like. The invention provides a support bandage comprising a strap having an inner end (3) and a main part (4) to be placed around a limb (2), and an outer end to be secured on the outer surface of the strap. The outer end is split into a main tab (6) which is slideable on the strap near the outer end of the main part of the strap and to be secured on said outer surface of the strap, and an adjustment tab (8) connected to the main part of the strap and to be secured on the outer surface of the main tab, wherein the encircling circumference of the strap may be adjusted by releasing, adjusting and re-securing the position of the adjustment tab (8) relative to the main tab (6).

## Description

### Field of the invention

The present invention relates to a support bandage for treatment and pain relief of repetitive strain injuries (RSI), typically tennis elbow (epicondylitis) and the like. The bandage is provided with double fasteners enabling easy adaptation and optimal setting of the treatment pressure for maximum pain relief and comfort.

### State of the art

Support bandages for treating tennis elbows are previously known. For instance reference may be made to US patent No. 5,865,775 and US patent application publication No. US2003/0032912. Generally, the bandage comprises a sleeve encircling the forearm and a pressure is set by adjusting a fastener. The fastener usually comprises a buckle wherein a hook tab is fastened against a hook engageable loop surface. The hook and loop surfaces generally have a substantial width and length since the bandage should fit various arm sizes and should be able to withstand the counteracting forces from the arm muscles. A problem is that it is difficult to set a correct pressure for the patient himself, especially because the swelling of the arm may decrease because of the treatment or increase because of activity and flexing the muscles. Also, the patient can only use his left hand (for a right-handed patient the right arm is usually the injured one) and the patient should be able to set the treatment pressure himself because the maximum pain relief is only achieved in a very narrow range which varies with time and can only be felt by the patient himself.

### Brief summary of the invention

The object of the invention is to provide a support bandage with easy adjustment for the patient, even with one hand only. The object is achieved by providing a support bandage with one fastener securing the strap loosely in a basic position and with one further fastener enabling tightening and loosening of the strap.

The invention provides a support bandage comprising a strap having an inner end and a main part to be placed around a limb, and an outer end to be secured on the outer surface of the strap, characterised in that the outer end is split into a main tab which is slideable on the strap near the outer end of the main part of the strap and to be secured on said outer surface of the strap, and an adjustment tab connected to the main part of the strap and to be secured on the outer surface of the main tab, wherein the encircling circumference of the strap may be adjusted by releasing, adjusting and re-securing the position of the adjustment tab relative to the main tab.

The invention is defmed in the attached claim 1, while further developments are set forth in the dependent claims.

### Brief description of the drawings

The invention will be described in detail below with reference to the accompanying drawings in which:
Fig 1 is a perspective view of a bandage according to the invention applied on an arm,
Fig 2 is a perspective view of the bandage of Fig. 1 partly opened,
Fig 3 is a schematic side view of the bandage with the fastener components split apart for ease of explanation, and
Fig 4 is a perspective view of the bandage showing the fully opened lacing.

### Detailed description of preferred embodiments

As mentioned in the introduction this invention relates to a support bandage for the relief of epicondylitis, commonly known as tennis elbow or golfer's elbow. However, the bandage may also be applicable to other forms of tendonitis. The working principle involves applying suitable pressure on the muscle in question, commonly the bulkiest portion of the upper forearm. To obtain best pain relief the pressure should be adapted to the sensations of the patient. Pressure should be maximised during activity but the resting pressure should be low enough to minimise any risks of complications related to prolonged, continuous, pressure. The patient knows best when the correct pressure is set. The pressure may vary over time due to swelling of the arm. Thus, it is a great advantage if the patient himself can adjust the pressure with his free hand.

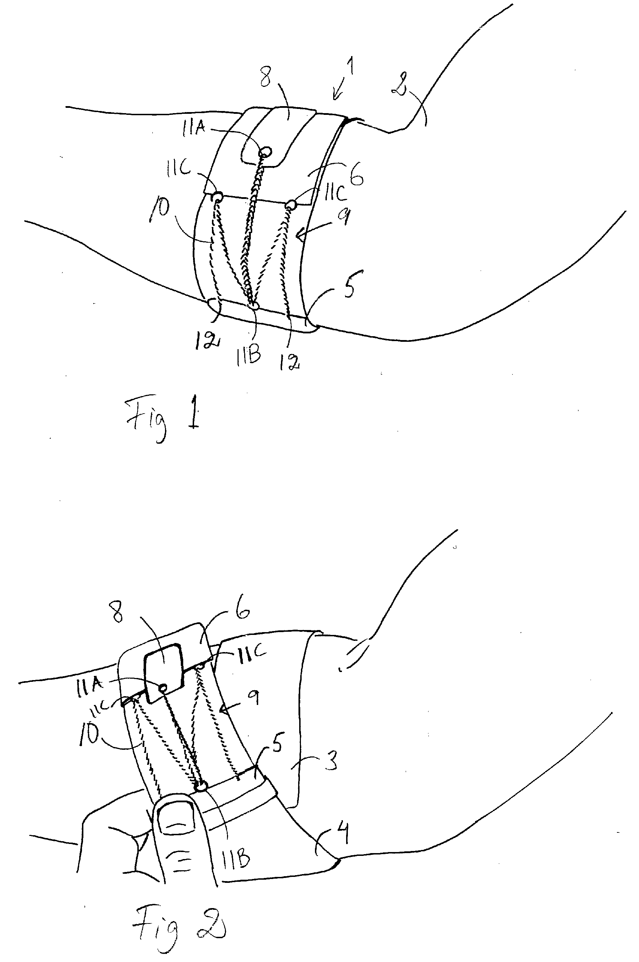

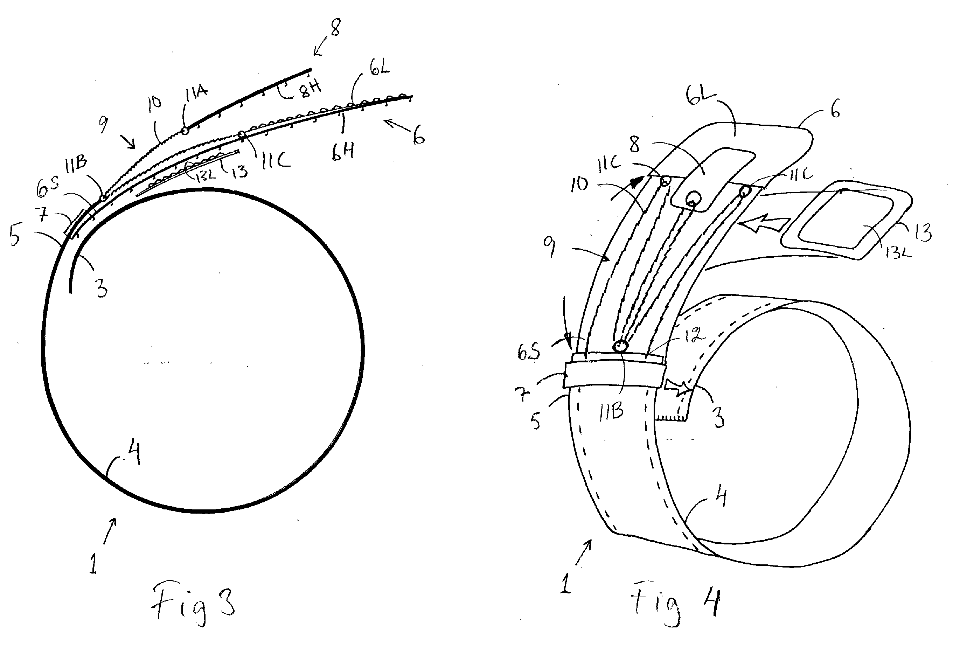

Figures 1 and 2 show a bandage 1 as applied to a right arm 2 of a patient. In Fig 2 the bandage is partly opened. The bandage consists mainly of a strap made of non-stretch, that is substantially inelastic, material of which the inner surface is applied against the arm. Decreasing the encircling circumference of the strap increases the pressure on the arm and vice versa. The strap has an inner end 3 and a main part 4 encircling the arm and ending at an outer end 5 of the main part 4. The outer end of the complete strap is split into two parts, a main tab 6 secured to the outer surface of the strap and an adjustment tab 8 in turn secured to the main tab 6. A lacing 9 connects the outer end 5 of the main part 4 to both the main tab 6 and the adjustment tab 8. The lacing 9 comprises a string 10 at both ends attached at points 12 to the outer end 5 and running through a ring 11A on the adjustment tab 8, one ring 11B at the centre of the end 5 and two rings 11C on the main tab 6. (The ring 11B can be replaced by two separate rings.)

The function of the support bandage 1 will be explained with reference to fig 3 in which the fasteners constituted by the main tab 6 and the adjustment tab 8 are shown separated in order to show more clearly the hook and loop surfaces. When the support bandage is applied on an arm in correct position a hook surface 8H on the inside of the adjustment tab 8 engages a hook engageable outer loop surface 6L of the main tab, and a hook surface 6H engages a hook engageable loop surface on the outside of the main strap.

When putting on the bandage the first time, the adjustment tab 8 and the main tab 6 are attached to each other and the main tab 6 is opened only, as is shown in Fig 2. The strap is wrapped around the arm starting from the inner end 3 and ending by attaching the main tab 6 to the strap. Because of the novel design it is not critical if the strap is a little loose. Thus, it is fairly simple to put on a bandage, even for the patient himself. This way of donning the bandage is typically only performed at the initial fitting.

Then, the patient should set the correct treatment pressure. This is done by releasing the adjustment tab 8 from the main tab 6 without releasing the main tab 6 from the underlying strap. This is also fairly easy to do, because the adjustment tab 8 is preferably made narrower than the main tab 6 and thus the securing force is lower. The patient may pinch the adjustment tab 8 with his thumb and forefinger while supporting his hand with the other fingers against the strap or arm. Then the patient can increase the pressure by pulling the adjustment tab 8. With this action the lacing 9 pulls the end 5 of the main part of the strap towards the main tab 6, thus decreasing the distance therebetween to take up any slack and then increasing the tension of the strap. The pressure is of course decreased by slackening the tension on the adjustment tab 8 in the opposite direction. As may be seen in fig 3, the outer end 5 is running freely through a loop 7 integral with the main tab 6. At this end the main tab 6 has a smooth surface 6S such that there is very little resistance when the end 5 is pulled through the loop 7. Even if the whole strap from the inner end 3 to the outer end 5 is made of hook engageable material on the outside, which is practical, the strap runs smoothly against the smooth surface 6S.

When the patient is satisfied with the pressure he attaches the adjustment tab 8 to the main tab 6.

With the design of the lacing as shown in the figures, with the string 10 running once between the end 5 and the main tab 6 and once between the end 5 and the adjustment tab 8, the lacing provides a mechanical advantage with a factor 2 like a double pulley mechanism. In other words, when moving the adjustment tab 8 a distance, the end 5 is moved half that distance, increasing the force between the end 5 and the main tab 6 compared to that which the patient pulls at the adjustment tab 8. The string is arranged in a symmetrical fashion in order to pull with the same force at both sides, thus avoiding that the bandage is pulled awry.

The fully opened lacing 9 is shown in figure 4. The outer end 5 of the main part just reaches through the loop 7 and about half of the adjustment tab 8 reaches the loop surface 6L of the main tab 6.

However, the lacing 9 is optional, and could be replaced with a single string or band between the end 5 and the adjustment tab 8, i.e. without the mechanical advantage. Even in this case the adjustment tab 8 is helpful, since it is easier to adjust the position of the small tab than the position of the larger main tab 6, the size of which is needed to give the proper stability.

Sometimes it is desired to get a more pronounced pressure at a certain area. For this reason a pressure plate 13 as shown in Figs. 3 and 4 may be inserted between the inside of the main tab 6 and the outside of the inner end 3 of the main part 4. The pressure plate 13 may be placed over a tendon medially or laterally. The pressure plate 13 may be provided in different sizes and hardnesses. The pressure plate may be slightly curved and flexible to fit around a section of the arm. The outer surface of the plate 13 is suitably provided with a hook engageable loop surface 13L to be fastened against the part of the inner surface 6H of the main tab 6 where the plate is to be attached. As an alternative, a pocket (not shown) may be provided on the inside of the main tab 6 for accommodating the pressure plate 13 securely.

The bandage may be manufactured in various materials as is known in the art. Preferably, the materials are laminate materials with proper hook surfaces and hook engageable/loop surfaces as required.

The present invention provides a support bandage for treating epicondylitis and repetitive strain injuries, having several advantages over the prior art. Because of the arrangement with the two fastening tabs the bandage is easy to put on and take off, even with one hand by the patient himself. Only at the initial fitting the main tab is opened and closed loosely around the arm. For the daily doffing and donning and for fine adjustment the adjustment tab is used. Thanks to the mechanical advantage of the lacing the patient may adjust the pressure easily and with less force. The scope of the invention is only limited by the claims below.

## Claims

1. A support bandage (1) comprising a strap having an inner end (3) and a main part (4) to be placed around a limb (2), and an outer end to be secured on the outer surface of the strap, **characterised in that** the outer end is split into a main tab (6) which is slidable on the strap near the outer end (5) of the main part of the strap and to be secured on said outer surface of the strap, and an adjustment tab (8) connected to the main part of the strap and to be secured on the outer surface of the main tab (6), wherein the encircling circumference of the strap may be adjusted by releasing, adjusting and re-securing the position of the adjustment tab (8) relative to the main tab (6).

2. A support bandage according to claim 1, **characterised in that** the adjustment tab (8) is connected to the main part of the strap by means of a lacing (9).

3. A support bandage according to claim 2, **characterised in that** the lacing (9) provides a mechanical advantage.

4. A support bandage according to claim 3, **characterised in that** the mechanical advantage is 1:2.

5. A support bandage according to any one of the preceding claims, **characterised in that** an inner surface of the adjustment tab (8) is provided with a hook surface (8H) to be secured on a loop surface (6L) arranged on the outer surface of the main tab (6).

6. A support bandage according to claim 5, **characterised in that** an inner surface of the main tab (6) is provided with a hook surface (6H) to be secured on a loop surface arranged on the outer surface of the strap (3, 4).

7. A support bandage according to any one of the preceding claims, **characterised in that** the adjustment tab (8) is narrower than the main tab (6).

8. A support bandage according to any one of the preceding claims, **characterised in that** a pressure plate (13) is placed inside the main tab (6) and outside the main part of the strap.

9. A support bandage according to claim 8, **characterised in that** the outer surface of the plate (13) is provided with a hook engageable loop surface (13L) to be fastened against the inner surface (6H) of the main tab (6).

10. A support bandage according to claim 8, **characterised in that** the pressure plate (13) is placed in a pocket arranged on the inside of the main tab (6).
